# EUROPEAN PATENT SPECIFICATION

(11) **EP 3 911 941 B1**
(45) Date of publication and mention of the grant of the patent: **12.03.2025**
(21) Application number: 20741925.0
(22) Date of filing: 17.01.2020
(51) Int. Cl.: G01N 21/64, G01N 21/27, G01N 21/63

(54) **TISSUE PHANTOMS**
GEWEBEPHANTOME
FANTÔMES DE TISSU

(30) Priority: 17.01.2019 US 201962793839 P
(43) Date of publication of application: 24.11.2021
(73) Proprietor: University Health Network, Toronto, Ontario M5G 2C4 (CA)
(72) Inventor: DACOSTA, Ralph, Toronto, Ontario M5G 2C4 (CA); OTTOLINO-PERRY, Kathryn, Etobicoke, Ontario M5G 2C4 (CA); GIBSON, Christopher, Toronto, Ontario M5G 2C4 (CA); ANANTHA, Nayana Thalanki, Toronto, Ontario M5G 2C4 (CA); O'BRIEN, Cristiana, Toronto, Ontario M5G 2C4 (CA)
(74) Representative: PGA S.p.A., Milano, Succursale di Lugano
(86) International application number: PCT/IB2020/050379
(87) International publication number: WO 2020/148720

(56) References cited:
- EP-A1- 1 532 431
- CA-A1- 2 478 083
- CA-A1- 2 869 899
- CA-A1- 2 891 990
- US-A1- 2005 187 478
- US-A1- 2005 187 478
- US-A1- 2016 370 285
- US-B2- 10 024 785
- US-B2- 6 441 892
- US-B2- 8 894 637
- US-B2- 9 042 967
- HARRIS E J ET AL: "Evaluation of a novel low light level (L3 vision) CCD technology for application to diffraction enhanced breast imaging", NUCLEAR INSTRUMENTS & METHODS IN PHYSICS RESEARCH. SECTION A, ELSEVIER BV * NORTH-HOLLAND, NL, vol. 513, no. 1-2, 1 November 2003 (2003-11-01), pages 27 - 31, XP004470072, ISSN: 0168-9002, DOI: 10.1016/S0168-9002(03)02130-2
- RICK PLEIJHUIS ET AL: "Tissue-simulating Phantoms for Assessing Potential Near-infrared Fluorescence Imaging Applications in Breast Cancer Surgery", JOURNAL OF VISUALIZED EXPERIMENTS, no. 91, 19 September 2014 (2014-09-19), XP055725724, DOI: 10.3791/51776
- JONATHAN P. CELLI ET AL: "Imaging and Photodynamic Therapy: Mechanisms, Monitoring, and Optimization", CHEMICAL REVIEWS, vol. 110, no. 5, 12 May 2010 (2010-05-12), US, pages 2795 - 2838, XP055400114, ISSN: 0009-2665, DOI: 10.1021/cr900300p
- TUCKER MATTHEW B ET AL: "Creation of an optically tunable, solid tissue phantom for use in cancer detection", PROGRESS IN BIOMEDICAL OPTICS AND IMAGING, SPIE - INTERNATIONAL SOCIETY FOR OPTICAL ENGINEERING, BELLINGHAM, WA, US, vol. 10480, 8 February 2018 (2018-02-08), pages 104800H - 104800H, XP060101852, ISSN: 1605-7422, ISBN: 978-1-5106-0027-0, DOI: 10.1117/12.2290499
- SLAVINE ET AL.: "Construction, calibration and evaluation of a tissue phantom with reproducible optical properties for investigations in light emission tomography", IEEE DALLAS ENGINEERING IN MEDICINE AND BIOLOGY WORKSHOP, 11 November 2007 (2007-11-11), XP031232139
- LEAVESLY ET AL.: "A calibrated tissue phantom for small animal fluorescence imaging - art. no. 68700C", PROCEEDINGS OF SPIE - THE INTERNATIONAL SOCIETY FOR OPTICAL ENGINEERING, February 2008 (2008-02-01), XP055725728
- VAN VEEN ET AL.: "Intraoperatively assessed optical properties of malignant and healthy breast tissue used to determine the optimum wavelength of contrast for optical mammography", JOURNAL OF BIOMEDICAL OPTICS, vol. 9, no. 6, November 2004 (2004-11-01), pages 1129 - 36, XP055725726
- PLEIJHUIS ET AL.: "Tissue-simulating Phantoms for Assessing Potential Near- infrared Fluorescence Imaging Applications in Breast Cancer Surgery", JOURNAL OF VISUALIZED EXPERIMENTS, 19 September 2014 (2014-09-19), pages 1 - 6, XP055725724
- SOLOMON ET AL.: "Optical Imaging in Cancer Research : Basic Principles, Tumor Detection, and Therapeutic Monitoring", MEDICAL PRINCIPLES AND PRACTICE, 2011, pages 397 - 415, XP055725722
- HAJ-HOSSEINI ET AL.: "Development and characterization of a brain tumor mimicking fluorescence phantom", INTERNATIONAL SOCIETY FOR OPTICAL ENGINEERING, 2014, pages 1 - 6, XP060033804

## Description

### TECHNICAL FIELD

The present disclosure relates to a tissue phantom, and in one exemplary embodiment, a breast tissue phantom. The phantom may be used to calibrate an imaging device that is used to identify one or more of residual cancer cells, precancerous cells, and satellite lesions in a surgical site such as a surgical cavity or in a tissue specimen removed from a surgical cavity, such as breast tissue removed during Breast Conserving Surgery ("BCS"). In addition, the disclosed tissue phantoms may be used to train or educate surgeons (or others) to identify one or more of residual cancer cells, precancerous cells, and satellite lesions in a surgical site that become visible in a surgical site or in an excised tissue specimen through use of an imaging device. The device also may be used to identify potential interactions between different types of imaging and/or contrast agents used together during surgery, such as BCS, to make residual cancer cells, precancerous cells, satellite lesions, and/or other malignant cells in surgical cavities, tissue specimens, lymph nodes, or other areas visible to the surgeon.

### INTRODUCTION

Surgery is one of the oldest types of cancer therapy and is an effective treatment for many types of cancer. Oncology surgery may take different forms, dependent upon the goals of the surgery. For example, oncology surgery may include biopsies to diagnose or determine a type or stage of cancer, tumor removal to remove some or all of a tumor or cancerous tissue, exploratory surgery to locate or identify a tumor or cancerous tissue, debulking surgery to reduce the size of or remove as much of a tumor as possible without adversely affecting other body structures, and palliative surgery to address conditions caused by a tumor such as pain or pressure on body organs.

In surgeries in which the goal is to remove the tumor(s) or cancerous tissue, surgeons often face uncertainty in determining if all cancer has been removed. The surgical bed, or tissue bed, from which a tumor is removed, may contain residual cancer cells, i.e., cancer cells that remain in the surgical margin of the area from which the tumor is removed. If these residual cancer cells remain in the body, the likelihood of recurrence and metastasis increases. Often, the suspected presence of the residual cancer cells, based on examination of surgical margins of the excised tissue during pathological analysis of the tumor, leads to a secondary surgery to remove additional tissue from the surgical margin.

For example, breast cancer, the most prevalent cancer in women, is commonly treated by breast conservation surgery (BCS), e.g., a lumpectomy, which removes the tumor while leaving as much healthy breast tissue as possible. Treatment efficacy of BCS depends on the complete removal of malignant tissue while leaving enough healthy breast tissue to ensure adequate breast reconstruction, which may be poor if too much breast tissue is removed. Traditionally, tumor margins are visualized under standard white light (WL) in an operating room in order to determine the effectiveness of the BCS procedure.

Imaging devices may also be used to evaluate specimen tissue for the presence of cancer cells. For example, imaging devices may be used to determine the amount of cancer cells, if any, remaining after a BCS procedure, thus determining the efficacy of the procedure. Imaging devices may also be used to provide guidance during the BCS procedure. Calibration of the imaging devices, as well as training and/or education of the persons using the imaging devices to identify residual cancer cells will contribute to the efficacy of removing residual cancer cells during BCS.

Scientific paper titled "Evaluation of a novel low light level (L3 vision) CCD technology for application to diffraction enhanced breast imaging", E.J. Harris et al., published by "Nuclear Instruments and Methods in Physics Research", A 513 (2003) 27-31, is directed to evaluate the potential of low-light level CCD for scanning human breast tissue phantoms.

Scientific paper titled *"*Tissue-simulating Phantoms for Assessing Potential Near-infrared Fluorescence Imaging Applications in Breast Cancer Surgery", Rick Pleijhuis et al., published by "Journal of Visualized Experiments", discloses a tissue-simulating breast phantom, comprising fluorescent dye, having optical characteristics of both healthy and diseased breast tissues.

Scientific paper titled *"*Creation of an optically tunable, solid tissue phantom for use in cancer detection", Matthew B. Tucker et al., published in "Clinical and Translational Neurophotonics 2018", discloses a tissue phantom, comprising fluorescent material, that can be tuned in order to simulate cancerous or healthy tissue.

### SUMMARY

A tissue phantom according to the invention is disclosed in any of claims 1-15.

The present disclosure may demonstrate one or more of the above-mentioned desirable features. Other features and/or advantages may become apparent from the description that follows.

In accordance with one aspect of the present disclosure, a tissue phantom includes a first portion having the optical properties of healthy tissue and a second portion having the optical properties of cancerous tissue.

In accordance with another aspect of the present disclosure, a method of calibrating an optical instrument. The method including illuminating a tissue phantom with excitation light from the optical instrument, detecting optical emissions emitted by the tissue phantom in response to illumination with the excitation light, and calibrating the optical instrument based upon the detected fluorescence.

### BRIEF DESCRIPTION OF THE DRAWINGS

The present disclosure can be understood from the following detailed description either alone or together with the accompanying drawings. The drawings are included to provide a further understanding of the disclosed teachings and are incorporated in and constitute a part of this specification. The drawings illustrate one or more example embodiments of the present disclosure and together with the description serve to explain various principles and operations.
FIG. 1A is an illustration of the conversion of ALA to PpIX in a tumor cell;
FIG. 1B shows peak absorption and emission for PpIX;
FIGS. 2A - 2D show components of a first embodiment of a tissue phantom including a first embodiment of a diseased tissue portion of the tissue phantom in accordance with the present disclosure;
FIGS. 3A and 3B show images of the tissue phantom of FIGS. 2A-2D in use;
FIG. 3C is an example of a dial to be used to cover the wells shown in FIGS. 2B and 3B;
FIG. 4 is an image of a second embodiment of a diseased portion to be used with the tissue phantom of FIGS. 2A-2D in accordance with the present disclosure;
FIGS. 5A and 5B are images of a third embodiment of a diseased portion to be used with the tissue phantom of FIGS. 2A-2D in accordance with the present disclosure;
FIG. 6 is an image of a fourth embodiment of a diseased portion to be used with the tissue phantom of FIGS. 2A-2D in accordance with the present disclosure;
FIGS. 7 and 8 are images of a second embodiment of a tissue phantom in accordance with the present disclosure;
FIG.9 is an image of a third embodiment of a tissue phantom in accordance with the present disclosure;
FIG. 10 is an image of a fourth embodiment of a tissue phantom in accordance with the present disclosure;
FIG. 11 is a flowchart illustrating an example method for making the tissue phantom shown in FIG. 10;
FIG. 12 is an image of a fifth embodiment of a tissue phantom in accordance with the present disclosure;
FIG. 13 is an image of a lymph node containing Methylene Blue (MB);
FIG. 14 shows peak absorption and emission for MB;
FIGS. 15 and 16 are images of a first embodiment of a MB tissue phantom in accordance with the present disclosure;
FIGS. 17 and 18 show images of a second embodiment of a MB tissue phantom in accordance with the present disclosure;
FIG. 19 is an image of a third embodiment of a MB tissue phantom in accordance with the present disclosure;
FIGS. 20A and 20B are images of a thin film tissue phantom that may be used with the MB tissue phantom of FIG. 19;

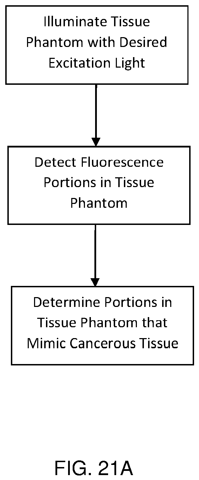

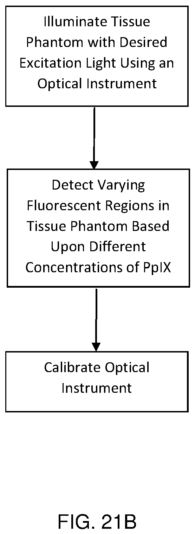

FIGS. 21A and 21B are flowcharts illustrating example methods of using a tissue phantom in accordance with the present disclosure.

### DESCRIPTION OF VARIOUS EXAMPLE EMBODIMENTS

Tissue phantoms, as discussed herein, may be used to calibrate an imaging device and/or to provide practice for an operating surgeon. For example, the tissue phantoms may represent and mimic the optical properties of "normal" or "healthy" tissue. Additionally, the tissue phantoms may include one or more portions that represent and mimic the optical properties of diseased or abnormal tissue such as "cancerous" tissue.

In alternative embodiments, the tissue phantom may be configured to include more than one component of a tissue and can be used to calibrate an imaging device or other device configured to distinguish one tissue component from another or locate one tissue component relative to another. In addition to calibration, the phantom can also be used for training purposes. For example, the phantom may be configured to represent human breast tissue and may contain different components/tissue types such as adipose tissue, connective tissue, and vasculature and the phantom can be used to train surgeons to locate the blood or vasculature relative to the adipose or connective tissue with an imaging device.

In another example, the tissue phantom may be used to train new users of a fluorescence imaging device to correctly identify tissues based on their fluorescence (e.g., identifying red tumor against green/pink connective/adipose tissue background). Or to show how certain tissues would fluoresce when imaged with such a device (blood shows up dark red/black for example, which may not be intuitive to new users).

As noted above, in accordance with one aspect of the present disclosure, the tissue phantom is configured to include a "normal" or "healthy" tissue portion and one or more "diseased" or "abnormal" tissue portions. The healthy tissue of the tissue phantom can be any type of tissue and the diseased tissue or "target" tissue of the tissue phantom can be chosen to mimic any disease found in the particular type of healthy tissue modeled by the tissue phantom. The examples provided herein discuss a breast tissue phantom having one or more areas of diseased tissue, i.e., cancerous tissue or tumors. It should be understood that these examples are non-limiting examples only and that the concept of a tissue phantom comprising healthy and diseased tissue is applicable to many other types of human and animal tissues and their diseases. Although discussed herein with regard to breast tissue, it is possible to use the present disclosure as a guide to create a tissue phantom representative of any tissue having a disease based on the optical properties for normal tissue and diseased tissue for the particular tissue and disease of interest. For example, knowing the absorption coefficient and reduced scattering coefficient of the chosen tissue type (for both normal and diseased tissues) at the wavelength that is being used for excitation would permit the creation of a phantom for a particular tissue having a particular disease as described herein. To create such a custom tissue phantom, information regarding how the tissue appears when imaged using a particular excitation light source and optical filter combination would be relied upon. For example, a fluorescence emission spectrum and/or fluorescence images of the tissues would provide the information needed. Examples of diseased tissue that have optical properties that may differ from the optical properties of healthy tissue include inflamed tissue (e.g., rheumatoid arthritis), fibrotic tissue, and ischemic tissue.

For example, in accordance with the present disclosure, tissue phantoms representative of healthy or diseased tissues that may be created in as disclosed herein may include: spinal cord, brain, skin, limbs (sarcoma), oral cavity, prostate, cervix, colon, thyroid, ovaries, lymph nodes, lungs, pancreas, esophagus, muscle, bone, cartilage, uterus, or vagina. This list is intended to provide examples only and is not intended to limit the range of possible phantoms created in accordance with the present disclosure. In exemplary embodiments of a phantom configured to represent healthy tissue and abnormal or diseased tissue such as "cancerous tissue," the "cancerous tissue" of the phantom contains a material that will cause the "cancerous tissue" of the phantom, when illuminated with excitation light having a known wavelength, to fluoresce or emit light having a wavelength which will allow detection/visualization of the "cancerous tissue" relative to the healthy tissue. For example, in some embodiments, a tissue phantom in accordance with the present disclosure may comprise "healthy tissue" configured to fluoresce green when illuminated with excitation light having a wavelength of between about 400 nm and about 450 nm. In addition, the tissue phantom may comprise "cancerous tissue" configured to fluoresce red when illuminated with the same excitation light having a wavelength of between about 400 nm and about 450 nm. An example of the material included in the "cancerous tissue" of the phantom that fluoresces a red color when illuminated with excitation light having a wavelength between about 400 nm and about 450 nm is the porphyrin PpIX. Alternatively, other fluorophores can be used to represent tissues that are different from healthy tissue, also referred to herein as "target tissue.'" For example, indocyanine green (ICG), a green dye such as Pacific Green (https://www.thermofisher.com/ca/en/home/life-science/cell-analysis/fluorophores/pacific-green-dye.html), IRDye 800CW, or other fluorophores of interest may be used. In an example embodiment where blood or vasculature is the "target tissue," ICG may be used in the portion of the phantom that represents the blood or vasculature.

In the example breast tissue phantoms disclosed herein, PpIX has been selected as the fluorophore of interest. PpIX is a fluorescent molecule that, when excited by the appropriate excitation light, emits a red fluorescence. The PpIX molecule is naturally broken down by healthy tissue (non-cancerous tissue) in a patient to Heme. Thus, healthy tissue does not contain PpIX and therefore does not emit the red fluorescence. However, cancerous tissue is not able to process PpIX and, thus, the PpIX collects in the cancerous tissue. The PpIX collected in cancer cells, when excited by light emitted by an imaging device and having a wavelength of between about 400 nm and about 450 nm, fluoresces red, making the cancerous tissue appear red to the imaging device. This allows a user of the imaging device to determine the presence or absence of cancerous cells based upon the corresponding presence or absence of red fluorescence emitted by the PpIX molecules.

As disclosed herein, a tissue phantom may be used with an imaging device in order to determine the presence, location and/or amount of the "cancerous" tissue with respect to the "normal" tissue within the tissue phantom. Such results may then allow a user to calibrate the imaging device, if the concentration of PpIX within the tissue phantom is known by the user.

Exemplary devices, systems, and methods for detecting cancer cells containing PpIX or other induced porphyrins during surgical intervention are disclosed in U.S. Provisional Patent Application No. 62/625,983, filed February 3, 2018 and entitled "Devices, Systems, and Methods for Tumor Visualization and Removal," and in PCT/CA2019/000015, filed February 1, 2019, entitled "Devices, Systems, and Methods for Tumor Visualization and Removal" and published as WO2019/148,268 on August 8, 2019,

During use of the tissue phantom for calibration of an imaging device, the imaging device may be inserted at least partially within a tissue phantom, such as a breast tissue phantom in accordance with the present disclosure and emit a desired wavelength of light to illuminate the tissue phantom. Illumination with the excitation light causes the "cancerous tissue" within the tissue phantom to fluoresce, as described above, thus making the cancerous tissue of the tissue phantom visible to the imaging device and to those observing the output of the imaging device. As discussed above, the "cancerous" tissue within the tissue phantom may fluoresce due to the presence of PpIX (or another fluorescent dye) contained within portions of the phantom.

The "normal" or "healthy" tissue of the tissue phantom does not include PpIX (or another fluorophore) and, therefore, does not fluoresce in the same manner, i.e., does not emit/reflect light at the same wavelength as the "cancerous tissue" when illuminated by the excitation light of the imaging device. However, the "normal" tissue of the tissue phantom is created to mimic normal healthy tissue, which autofluoresces when illuminated with the excitation light. Different healthy tissues emit different wavelengths of light in response to illumination by excitation light. Thus, when illuminating a tissue phantom with excitation light as disclosed herein, the different components of the phantom (healthy tissue, cancerous tissue) will emit different wavelengths of light in response. This allows the light emitted from the cancerous tissue to be distinguished from the light emitted by the healthy tissue of the tissue phantom and, thus, permits the surgeon to identify the presence of cancerous tissue and its location.

For example, for calibration of an imaging device configured to emit excitation light of between about 400 nm - 450 nm, the tissue phantom has optical properties that allow the phantom to mimic the emission response of tissue illuminated with excitation light of between about 400 nm - 450 nm. The optical properties of the tissue phantom can be narrowly tailored to mimic tissue response (of both healthy tissue and diseased tissue) to excitation by any range of excitation light. For example, the phantom can be formed to have optical properties that allow it to mimic tissue response to illumination by excitation light in the ultraviolet/blue range, near infrared range, and infrared range. For example, the present disclosure contemplates a tissue phantom having optical properties that mimic tissue response to illumination by excitation light in the following exemplary ranges: about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, about 900 nm - about 950 nm, about 950 nm - about 1000 nm, and/or various combinations therefor. In certain non-limiting, exemplary embodiments disclosed herein, the tissue phantom is configured to respond to illumination with excitation light in the blue/violent range, for example 405 nm, in a manner the same or substantially the same as human or animal tissue.

The tissue phantoms disclosed herein also can be used to help identify an optimum amount of PpIX to be collected in cancerous cells in order for the fluorescence of the cancer cells to be detected by the imaging device and/or the surgeon. Using this information, it is possible to then determine the appropriate amount or dose of porphyrin-inducing composition that should be administered to the patient, for example prior to BCS, as well as the timing of the dosage. For example, as disclosed in U.S. Provisional Patent Application No. 62/625,967, filed February 2, 2018 and entitled "Devices, Systems, and Methods for Tumor Visualization and Removal," and in U.S. Provisional Patent Application No. 62/625,983, filed February 3, 2018 and entitled "Devices, Systems, and Methods for Tumor Visualization and Removal," and in PCT/CA2019/000015, filed February 1, 2019, entitled "Devices, Systems, and Methods for Tumor Visualization and Removal" and published as WO2019/148,268 on August 8, 2019, the surgical subject or patient may be given a diagnostic dose (i.e., not a therapeutic dose) of a compound (imaging/contrast agent) such as the pro-drug aminolevulinic acid (ALA). As understood by those of ordinary skill in the art, dosages of ALA less than 60 mg/kg are generally considered diagnostic while dosages greater than 60 mg/kg are generally considered therapeutic. As disclosed herein, the diagnostic dosage of ALA may be greater than 0 mg/kg and less than 60 kg/mg, between about 10 mg/kg and about 50 mg/kg, between about 20 mg/kg and 40 mg/kg, and may be administered to the subject in a dosage of 5 mg/kg, 10 mg/kg, 15 kg/mg, 20 mg/kg, 25 mg/kg, 30 mg/kg, 35 mg/kg, 40 mg/kg, 45 mg/kg, 50 mg/kg, or 55 mg/kg. The ALA may be administered orally, intravenously, via aerosol, via immersion, via lavage, and/or topically. Although a diagnostic dosage is contemplated for visualization of the residual cancer cells, precancer cells, and satellite lesions, it is within the scope of the present disclosure to use the disclosed devices, systems, and methods to provide guidance during treatment and/or removal of these cells and/or lesions.

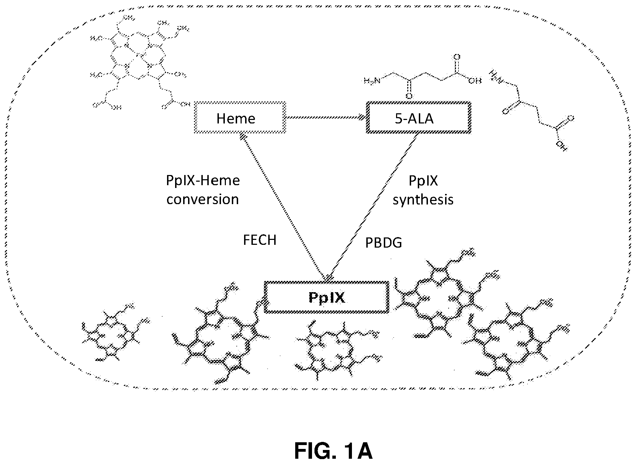

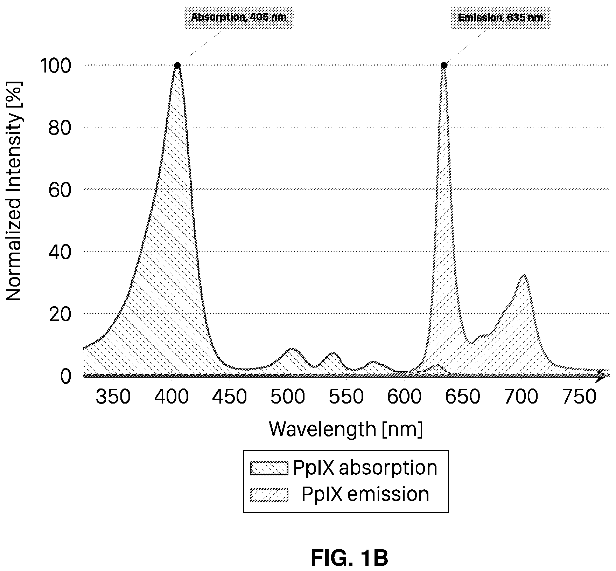

The ALA given to the patient induces porphyrin formation (protoporphyrin IX (PpIX)) in tumor/cancer cells present in the patient (FIG. 1A shows the conversion of ALA to PpIX within a tumor cell) and, when the cells containing PpIX are illuminated by the appropriate excitation light, an emission having a wavelength that appears as red fluorescence from cells containing the PpIX is captured by the imaging device. These cells are then visible against the green fluorescence emitted by the healthy tissues (which have broken down the PpIX into Heme and, thus, do not fluoresce a red color), which enhances the red-to-green fluorescence contrast between the tumor/cancer tissue cells and normal tissue cells (e.g., collagen) imaged with the device. ALA is non-fluorescent by itself, but PpIX emissions, when excited by excitation light having a wavelength of between 400 nm and about 450 nm, have wavelengths of about 630 nm, about 680 nm, and about 710 nm, with the 630 nm emission being the strongest. FIG. 1B illustrates the fluorescence emission of PpIX when excited with excitation light having a wavelength of 405 nm. Alternatively, the endogenous fluorescent difference between tumor/cancer cells or precancer cells and normal/healthy cells may be used without an imaging/contrast agent.

In exemplary embodiments, the non-activated, non-targeted compound configured to induce porphyrin in tumor/cancer cells, precancer cells, and/or satellite lesions is administered to a subject between about 15 minutes and about 6 hours before surgery, about 1 hour and about 5 hours before surgery, between about 2 hours and about 4 hours before surgery, or between about 2.5 hours and about 3.5 hours before surgery. These exemplary time frames allow sufficient time for the ALA to be converted to porphyrins in tumor/cancer cells, precancer cells, and/or satellite lesions. The ALA or other suitable compound may be administered orally, intravenously, via aerosol, via immersion, via lavage, and/or topically. Use of the tissue phantoms disclosed in the present application can determine at what concentration PpIX must be present in the phantom and, thus, the cancer cells of the subject administered the ALA, in order for the fluorescence of the cancer cells to be detected by the imaging device and/or the surgeon. Using this information, it is possible to then determine the appropriate amount of porphyrin-inducing composition that should be administered to the patient as well as the timing of the dosage, as discussed above.

The tissue phantoms disclosed herein are configured to have the same optical properties as human tissue that contains the target tissue or disease, such as cancer. Thus, for example, in the case of breast cancer, the tissue phantom may include a first portion that has the same optical properties as healthy skin/normal tissue, such as breast tissue. The tissue phantom also includes one or more second portions having the same optical properties as the diseased tissue, such as cancerous breast tissue. Thus, the breast tissue phantom "mimics" or is an on-the-bench representation of a human breast with breast cancer.

The first portion of the tissue phantom may comprise any material that is sufficient to mimic the optical properties of "normal" or "healthy" tissue, i.e., tissue that is not cancerous, to provide the tissue phantom with optical characteristics substantially the same as the optical characteristics of normal tissue. In some embodiments, the first portion of the tissue phantom may be made from a mixture of tris buffer and gelatin. The tris buffer aids to maintain the pH and stability of the tissue phantom, and the gelatin aids to provide a consistency that mimics an actual human or animal tissue. More specifically, the gelatin mixture allows the tissue phantom to cure into a long-lasting, reproducible shape that mimics the connective tissue found in human breast tissue. In other embodiments, the first portion of the tissue phantom may be formed of silicone, agar, or organic tissues from animals including chicken, pork, or beef.

Additionally, the first portion of the tissue phantom may include other components such as, for example, elements representative of hemoglobin and intralipid molecules. As discussed further below, the concentrations of the hemoglobin and intralipid molecules may be manipulated to adjust the optical properties of the tissue phantom such as scattering and absorption coefficients, isotropy, and/or turbidity. Sodium azide may also be added to the tissue phantom to provide an antibacterial agent that inhibits the growth of bacteria on the tissue phantom, thus increasing its longevity. Furthermore, the antibacterial agent may also help ensure that any visible fluorescence is not due to the presence of bacteria on or in the tissue phantom. The first portion of the tissue phantom may be made of one or more materials. In one example embodiment, the first portion of the tissue phantom may be homogenous throughout such that the tissue phantom comprises no more than a 2% variation in a full spectrum reflectance measurement throughout the phantom.

In some embodiments, the optical properties of the first portion of the tissue phantom may be selected by varying hemoglobin and/or intralipid concentrations. Hemoglobin concentrations may be varied to adjust the rate of absorption of incoming photons from, for example, an excitation light source of an imaging device. Intralipid concentrations may be varied to adjust for scattering of incoming excitation and/or other light from the imaging device. Hemoglobin and/or intralipid concentrations may be adjusted so that the material(s) of the tissue phantom substantially match the absorption and emission coefficients of "normal" tissue, such as normal breast tissue.

In some embodiments, the concentration of hemoglobin may range from approximately 0-50 µm. When using the high end of this range, the tissue phantom may produce a visibly stronger red fluorescence, which may be used to mimic the optical properties of tissue when the hemoglobin absorbs less light. When using the low end of this range, the tissue phantom may produce a visibly weaker red fluorescence, which may be used to mimic the optical properties of tissue when the hemoglobin absorbs more light. In some embodiments, the concentration of intralipids may range from approximately 0-20%.

In one example embodiment, the tissue phantom includes a hemoglobin concentration of 2.40 µM and an intralipid concentration of 1.20% for use with an imaging device that emits excitation light at a wavelength of 405 nm. This concentration of hemoglobin is below the normal concentration of hemoglobin in breast tissue, which typically ranges from 15-40 µM. The concentration of hemoglobin may be below the normal concentration because less hemoglobin is required in a tissue phantom (as compared with a patient's breast tissue) to match the desired absorption coefficient at 405 nm.

An example method for preparing tissue phantom material (healthy tissue material) is provided below:
Tris buffer
   - Suspend 6.1g of Tris and 8.8g NaCl in 800mL of RO water in a 1000mL Erlenmeyer flask.
   - Dissolve 1.0g of sodium azide into the same flask for a resultant pH of approximately 10.5.
   - Using pH indicator strips, adjust the pH to 7.4 by adding 5mL of hydrochloric acid in 1mL increments for a total of 5mL.
   - Adjust the total volume of the flask to 1000mL with RO water.
Tissue Phantom Material
   - Add 50g of gelatin 300 bloom to 500mL of Tris Buffer in a 1000mL flask.
   - Place the flask on a hot plate and heat until 50°C under constant stirring with a magnetic stir bar. Monitor the temperature with a thermometer or a thermal gun.
   - Once the gelatin is completely dissolved, remove the gelatin mixture from the heat source and cool 35°C on the lab bench under constant stirring.
   - Prepare 0.0872g of hemoglobin and 32ml of intralipid in a separate 25mL flask. Mix the hemoglobin into the intralipid until no clumping is visible.
   - Once cooled, stir the hemoglobin mixture into the gelatin.
   - Pour the gelatin mixture into an appropriate mould and set in the refrigerator for 24 h before removing.

In accordance with the present teachings, the second portion(s) of the tissue phantom may include material configured to represent diseased tissue. In various exemplary embodiments, the tissue phantom includes material configured to represent diseased tissue, e.g., cancer in breast tissue. The material configured to represent diseased tissue may be incorporated into the tissue phantom in various ways, positioned within the healthy tissue portion of the phantom or separated from the healthy tissue portion of the phantom. For example, in one embodiment, the material configured to represent the diseased tissue may be disposed sporadically throughout the tissue phantom. In other embodiments, the material configured to represent the diseased tissue may be disposed in one distinct location within the tissue phantom in order to represent, for example, a tumor. Each location of the diseased tissue within the tissue phantom may include portions with varying concentrations of diseased tissue. In some embodiments, the tissue phantom may be composed of 3D printed cells, for example cells or tissue that are printed into desired shapes with densities and optical properties that substantially match those of human tissue. In one example, the tissue phantom is a 3D printed ear.

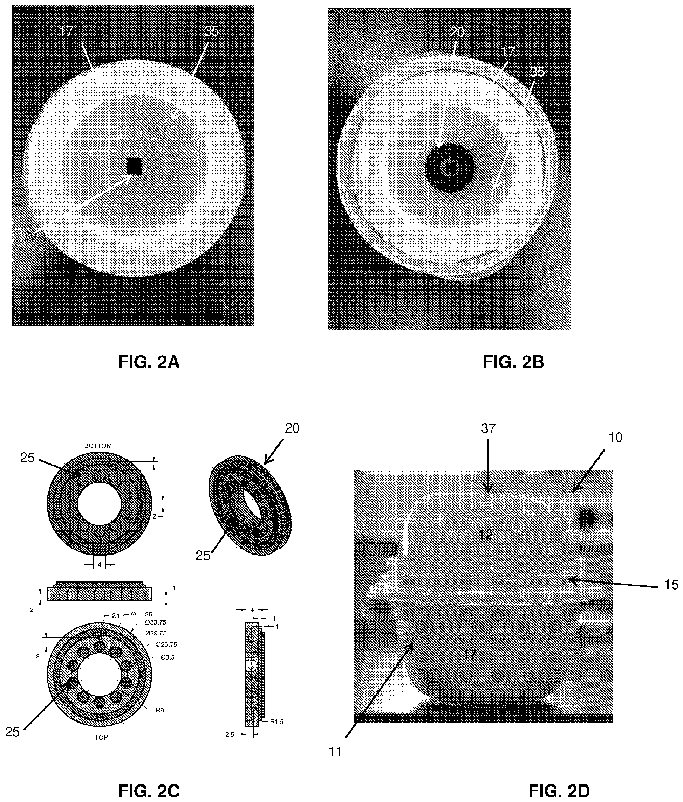

In accordance with the present disclosure, a tissue phantom includes a healthy tissue portion and a diseased tissue portion. In one example embodiment shown in FIG. 2D, a tissue phantom 10 includes a body 11 having a first member 12 and a second member 17. First member 12 and/or second member 17 may be hollow cylindrical or semi-spherical members with rounded outer edges. In the embodiment shown, each member 12, 17 has been molded to have a hollow interior. However, it is also contemplated that first and second members 12, 17 may be solid members. The first member 12 and second member 17 fit together to form body 11 of the tissue phantom 10 as shown in FIG. 2D and together form the first portion or healthy tissue portion of the tissue phantom 10. When first and second members 12, 17 are positioned together, a cavity 35 is formed within body 11. Tissue phantom 10 may be shaped and sized to represent a patient's breast. Tissue phantom may alternatively be embodied as excised breast tissue (i.e., representative of a lumpectomy specimen). In alternative embodiments, the tissue phantom may be shaped and sized to represent other body parts. Although discussed herein with regard human breasts and excised breast tissue, the phantoms of the present disclosure may be formed to replicate any tissue body part, human or animal.

First member 12 and second member 17 may be attached together at middle portion 15. In some embodiments, first member 12 and second member 17 are two distinct components. In other embodiments, first member 12 and second member 17 form one unitary component. First member 12 and second member 17 of phantom 10 may each comprise any material that is sufficient to mimic optical properties of "normal" or "healthy" tissue, i.e., tissue that is not cancerous, to provide the phantom with optical characteristics substantially the same as the optical characteristics of normal tissue. Thus, first member 12 and second member 17 may separately or together form a portion of the phantom 10 that has the optical properties of healthy tissue. In some embodiments, first member 12 and/or second member 17 may be formed of a mixture of tris buffer and gelatin. First member 12 and second member 17 may be formed of the same or different materials. In one example embodiment, the material(s) of first member 12 and second member 17 may be homogenous throughout such that the healthy tissue portion of the tissue phantom 10 comprises no more than a 2% variation in a full spectrum reflectance measurement throughout the phantom.

In accordance with the present disclosure, the tissue phantom 10 includes a diseased tissue or cancerous tissue portion. In the example embodiment of FIGS. 2A-2D, the material configured to represent the diseased tissue may form a part of the tissue phantom separate from the healthy tissue part of the phantom. For example, as shown in FIG. 2C, a tray 20 is configured to contain the material that represents the diseased tissue of the phantom. As shown in FIG. 2B, the tray 20 may be positioned in the cavity 35 defined by the body 11 of the phantom. In the example embodiment of FIG. 2A, the second member 17 of tissue phantom 10 may be configured to receive and support tray 20 and its contents, and tray 20 may be disposed within second member 17 of tissue phantom 10 as illustrated in FIG. 2B. Although illustrated as being positioned in the second member 17 of phantom 10, it is contemplated that tray 20 may be positioned in the first member 12 of the phantom 10, or two trays 20 may be provided, one in each member 12, 17 of the tissue phantom 10.

The tray may take on various shapes and configurations. An example configuration of tray 20 is illustrated in FIG. 2C. Tray 20 may have a rounded donut shape and may include one or more recesses 25 in a top surface thereof. Recesses 25 may be circular in shape or may have any other shape sufficient to hold the material configured to represent diseased tissue, which may be in solution form as discussed further below. Recesses 25 may be arranged uniformly around tray 20. Alternatively, recesses 25 may be arranged sporadically around tray 20. In one embodiment, tray 20 includes ten recesses 25.

Tray 20, including recesses 25, may be formed of a polymeric material, for example, polylactic acid (PLA). In some embodiments, tray 20 is coated, printed, and/or manufactured with a black paint, which acts an optical barrier between recesses 25 and second member 17 of tissue phantom 10. Thus, wavelengths emitted from an imaging device do not penetrate through recesses 25 and onto second member 17. These embodiments may allow for a more accurate determination of the optical properties detected in each recess 25, which may be used for calibration of an imaging device.

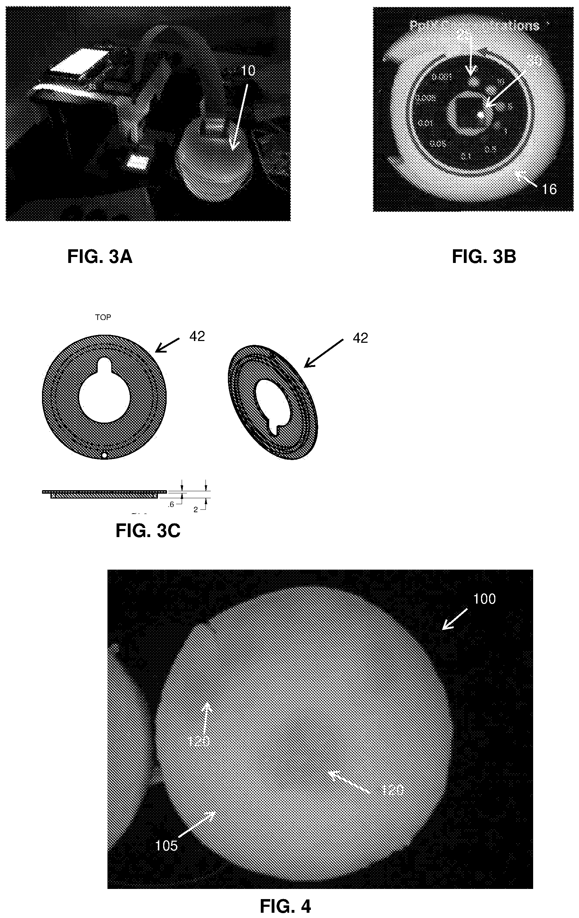

For calibration purposes, the size and location of recesses 25 may be dependent on their distance from the imaging device. In the example shown, each recess may have a diameter of 3.55 mm when the imaging device is intended to be disposed 2cm from tray 20 during testing/calibration. See, for example, FIG. 3A. FIG. 3B shows the tray 20 positioned in second member 17 of phantom 10 during fluorescent imaging. As will be understood by those in the art, in order to obtain reproducible results for calibration and training purposes, it is necessary to understand the distance between the excitation light source and the material configured to represent diseased tissue. It is further contemplated that the diameter of recesses 25 may increase as the imaging device is disposed further away from tray 20. Furthermore, each recess 25 may be the same size, or recesses 25 may be of varying sizes.

One or more materials may be disposed within each recess 25. The materials include a first composition configured to have the optical properties of diseased tissue. In addition, a second material configured to cause the diseased tissue material to fluoresce in response to illumination with excitation light is included. These materials may be in solid or liquid form, or a combination thereof. In accordance with one example embodiment, the first composition may include any material that is capable of mimicking the optical properties of "cancerous" tissue to provide one or more portions of the phantom with optical characteristics substantially the same as the optical characteristics of cancerous tissue. The first composition may include, for example, agar, phosphate buffered saline (PBS), water, agarose, dimethyl sulfoxide (DMSO) and/or blood tissue. These components may be used to hold the second material in suspension in the solution.

The second material may include PpIX and/or another fluorescent dye. Each recess 25 may include a solution with a different concentration of PpIX and/or fluorescent dye, such that the concentration of PpIX or fluorophore in each recess differs from that of an adjacent recess 25. For example, a first recess may be a control that does not contain any PpIX or dye. A second recess may contain a small concentration of PpIX (and/or another fluorescent dye), a third recess may contain a relatively greater concentration of PpIX (and/or another fluorescent dye), a fourth recess may contain an even greater concentration of PpIX (and/or another fluorescent dye), etc. In one example, tray 10 has ten recesses 25 such that each recess has one of the following concentrations of PpIX: 0.001 µM, 0.005 µM, 0.01 µM, 0.05 µM, 0.1 µM, 0.5 µM, 1 µM, 5 µM, 10 µM, and 20 µM.

The solutions disposed within each recess 25 may mimic the optical properties of "cancerous tissue." Thus, tray 20 may form a portion of tissue phantom 10 that has the optical properties of cancerous tissue.

The distal end of second member 17, where tray 20 is disposed, may include an opening 30 through which an optical power meter may be inserted into the tissue phantom. Opening 30, without an optical power meter, can be seen in FIGS. 2A and 2B. Opening 30 with an optical power meter positioned therein is shown in FIG. 3B. In some embodiments, opening 30 may be a 1 × 1 cm square hole. In other embodiments, opening 30 may be a round hole or any other shape sufficient for any well-known optical power meter.

An opening 37 may also be created in the end of first member 12 to allow access to the interior of the tissue phantom and the cavity 35 defined therein, to permit an imaging device a field of view of the diseased portion of the tissue phantom represented by the contents of the recesses in tray 20. An imaging device may be inserted at least partially through opening 37. Thus, opening 37 may be of sufficient size to receive at least a portion of an imaging device configured to be placed within a body lumen or cavity or surgical cavity. The imaging device may pass through opening 37 to access cavity 35 in any manner that maintains the integrity of tissue phantom 10 while providing the ability for the imaging device to illuminate the diseased tissue material in recesses 25 with excitation light and to receive emissions responsive to illumination by the excitation light from the diseased tissue material in recesses 25.

Once assembled, tissue phantom 10 includes first and second members 12, 17 attached together with tray 20 disposed within cavity 35 and positioned on second member 17. Additionally, a variety of solutions are disposed within each recess 25 of tray 20, such that each recess 25 has a different concentration of, for example, PpIX. An optical power meter may then be inserted at least partially through cavity 30, and an imaging device may be inserted at least partially through cavity 35. The imaging device, once inserted within tissue phantom 10, will then emit an excitation light onto and/or within tissue phantom 10. As discussed above, any recess 25 that contains PpIX (or another fluorescent dye) will be illuminated by the excitation light and emissions, in response to the excitation light, from the material in the recesses will be received and captured by the imaging device. Such may allow a user to determine which concentrations of PpIX are detectable by the imaging device. The diseased tissue material in the recesses 25 may be excited individually or in a group. To allow the material in each recess 25 to be illuminated with excitation light individually, a dial or cover 42 may be placed over the recesses 25 such that only a single recess is visible at a time. An example dial 42 is shown in FIG. 3C. An example of a plurality of recesses being illuminated at the same time is shown in FIG. 3B. The central square in the tissue phantom is an optical meter and the black surrounding the red recesses (or wells) is the material of the tray. This determination may then be used to calibrate the imaging device. The optical power meter inserted within cavity 30 measures the optical power of the imaging device.

The imaging device may emit white light (WL), fluorescence (FL), infrared (IR), or a mixture thereof. In some embodiments, the imaging device may emit excitation light at a wavelength from 350 nm to 450 nm and/or from 550 nm to 600 nm, and more specifically the imaging device may emit excitation light at a wavelength of 405 nm and/or 572 nm.

When illuminated with a wavelength of, for example, 405 nm, first member 12 and second member 17 will fluoresce in the same manner as healthy breast tissue, which autofluoresces under 405 nm excitation light and appears green in fluorescent images. Thus, the parts of the phantom 10 made to mimic healthy breast tissue will, for example, fluoresce green when illuminated with 405 nm excitation light. However, when the solutions within each recess 25 that contain PpIX are illuminated with a wavelength of, for example, 405 nm, the solutions will fluoresce in the same manner as cancerous breast tissue. Thus, the parts of the phantom 10 made to mimic cancerous tissue will, for example, fluoresce red when illuminated with 405 nm excitation light. This allows the cancerous tissue of the phantom to be distinguished from the healthy tissue of the phantom when both tissues are illuminated with the same excitation light, thus allowing a user to determine portions in tissue phantom that include the cancerous tissue.

Different concentrations of PpIX in each solution, will emit different wavelength intensities of light from tissue phantom 10. This allows the light emitted from each unique solution to be distinguished from the light emitted from a subsequent solution (with a different amount of PpIX). Additionally, this allows the light emitted from first and second end portions 12, 17 (the healthy tissue) to be distinguished from the light emitted from recesses 25 (the cancerous tissue). Therefore, a user can identify the presence of cancerous tissue and its location within tissue phantom 10.

Because the different concentrations of PpIX in each recess 25 emit different intensities of light from tissue phantom 10, a user may use tissue phantom 10 to calibrate an optical instrument. More specifically, an optical instrument may be used to illuminate tissue phantom 10 with excitation light, for example excitation light having a wavelength of 405 nm. As discussed above, a first solution disposed in a first recess 25 may have a first concentration of PpIX, and a second solution disposed in a second recess 25 may have a second concentration of PpIX. The first solution may emit a different intensity of light from the second solution when illuminated with the excitation light. A user may then calibrate the optical instrument by comparing the different concentrations of PpIX in the first and second solutions with the light emitted therefrom. Additionally or alternatively, a user may compare the amount of fluorescence from the first and second solutions with the concentrations of PpIX in order to calibrate the optical instrument.

The imaging device may also include a camera in order to capture the illuminated solution in recesses 25. Such results may then be displayed to a user.

In other embodiments of a tissue phantom, it may be desirable to remove the optical barrier provided by the tray 20, in order to visualize the "diseased tissue" or fluorophore representing the diseased tissue against the background of the "healthy tissue" forming the phantom 10. This may be accomplished in various ways such as, for example, placing the "diseased tissue" or fluorophore directly on the phantom material mimicking the healthy tissue, placing the "diseased tissue" or fluorophore on an optically clear surface (such as a microscope well slide) and then placing the clear surface on the phantom's "healthy tissue," or by creating wells in the phantom's "healthy tissue" and placing the "diseased tissue" or fluorophore in the wells.

In one example embodiment, shown in FIG. 4, the material forming the "diseased tissue," 120 e.g., a fluorophore, can be placed directly on the phantom material that mimics the healthy tissue 105, for example using a pipette, syringe, or paintbrush. The phantom material will absorb liquid fluorophores if placed directly on top. Alternatively, the fluorophore can be mixed with a solidifying agent such as gelatin to prevent this. A fluorescent image of a phantom 100 having healthy tissue portion 105 painted with a PpIX and gelatin mixture (red/pink) to form the diseased tissue portions 120 is shown in FIG. 4.

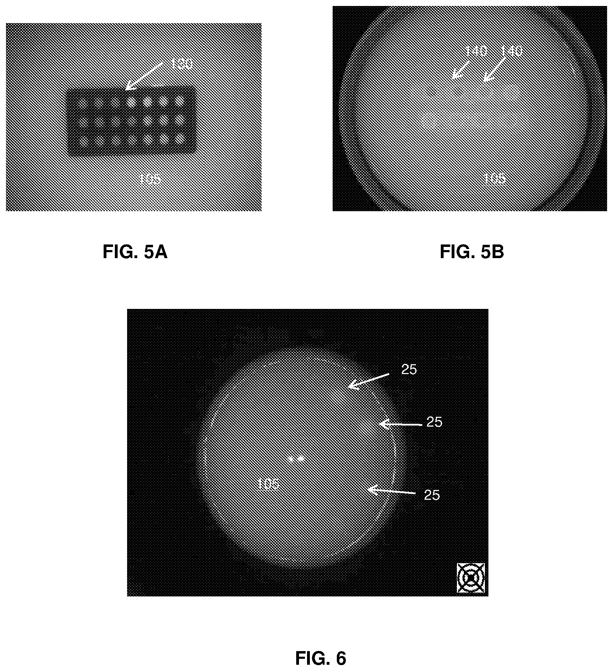

In another example embodiment, shown in FIGS. 5A and 5B, a surface or container with an optically clear bottom, such as a glass microscope well slide 130 (see FIG. 5A, containing PpIX in the right side of the top row) or plastic PCR tube lids 140 (see FIG. 5B, containing 630 nm quantum dots in decreasing concentration) is used to position the "diseased tissue" or fluorophore representing the diseased tissue onto the phantom material representing the healthy tissue. This allows for various concentrations of the fluorophore to be imaged against the green background (the phantom material forming the "healthy tissue" of the phantom will fluoresce green when illuminated with 405 nm excitation light) to see the contrast between each concentration of the fluorophore and the background. In another embodiment, tray 20 is transparent so that wavelengths of light emitted by a light source of the imaging device penetrate through recesses 25 and onto second member 17, i.e., against the healthy tissue of the phantom or the phantom background. These embodiments may provide a realistic scenario for training purposes by allowing a determination of the amount of "diseased tissue" required, or percentage of PpIX or fluorophore required, to visualize the diseased tissue in situ.

In the example embodiment shown in FIG. 6, recesses or wells can be created directly in the healthy tissue of the phantom material by cutting out cylindrical portions of the tissue phantom. This can be done, for example, using a punch biopsy tool (2-, 4-, 8-mm, or another diameter) to remove a cylindrical chunk of the phantom material. Then, the cylindrical recess or well can be filled with a fluorophore. The liquid fluorophore can be mixed with phantom material in liquid form and added to the well, to create a solid fluorescent well that will maintain its form over time. A phantom material concentration of greater than 50% will allow the mixture to solidify while not significantly affecting the fluorescent color of fluorophore mixed into the wells. With a stable fluorophore such as a quantum dot, this method can be used to create reproducibly fluorescent phantoms that maintain their fluorescence over time. FIG. 6 shows a fluorescent image of varying concentrations of PpIX mixed with 89% phantom material and pipetted into wells created with a 4 mm punch biopsy tool. This image was captured after the mixture solidified.

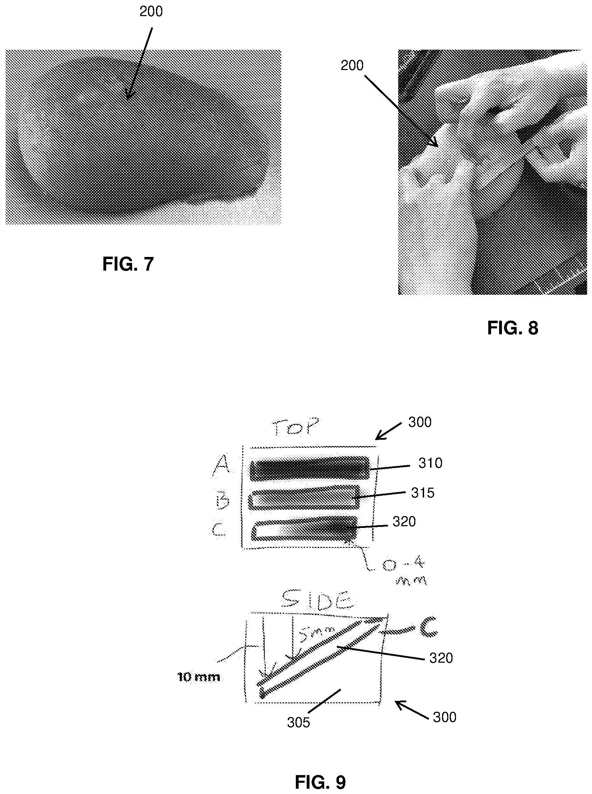

In some embodiments, as shown in FIGS. 7 and 8, a tissue phantom 200 may be used to mimic the shape of a patient's breast, which may include a breast lumpectomy cavity. Such phantoms are designed to mimic the shape and mechanical/elastic properties of a breast. These phantoms might come in a variety of different configurations.

In one example, the phantom may be formed as a full breast with a solid PpIX tumor inclusion. In this embodiment, a surgeon can palpate to locate the tumor as they would normally do prior to a surgery. The surgeon would then create an incision/surgical cavity accordingly. The surgeon would image the resected mass and the cavity to ensure clear margins.

In another example, the phantom may be formed as a full breast with a pre-made cavity containing a lumpectomy sample. Such an embodiment of the breast phantom would have a pre-made cavity and corresponding lumpectomy sample, both with positive margins (i.e., both including diseased or "cancerous" tissue that a surgeon could image). The cavity size and shape would be designed to allow the surgeon to manipulate an imaging device in accordance with the present disclosure in a variety of ways. This would allow surgeons to practice imaging at the bottom and sides of a surgical cavity, as well as under (premade) skin flaps.

As discussed above, the target tissue may be made identifiable by use a fluorophore. Although the example embodiments describe the use of PpIX, other fluorophores may be used. For example, a phantom in accordance with FIG. 7 can be combined with ICG. These models would train the surgeon to image in fluorescent (FL) mode and infrared (IR) mode, as well as to switch between them and observe the resulting images in real time. Fluorescent ICG "bars" similar to the ICG phantom would be placed within the cavity to mimic vasculature, both at and below the surface of the phantom.

In one example embodiment, a tissue phantom may include a first healthy tissue portion 305 and one or more target tissue portions 310, 315, 320. In the example shown in FIG. 9, the tissue phantom 300 includes solid ICG "bars" 310, 315, 320 to mimic vasculature or masses (target tissue) that contain ICG. The tissue phantom 300 may be a flat slab of the phantom tissue material with three ICG bars placed within: (A) a bar 310 directly at the top surface of the phantom 300, (B) a bar 315 submerged 3 mm below the surface of the phantom 300, and (C) a bar 320 on an angle such that one side of the bar is at the surface of the tissue phantom 300 and the other side of the bar is submerged 1 cm in the phantom material. This third bar 300 is also placed close to the end of the phantom 300 such that the slope of the bar can be visualized if the side of the phantom is imaged under IR excitation.

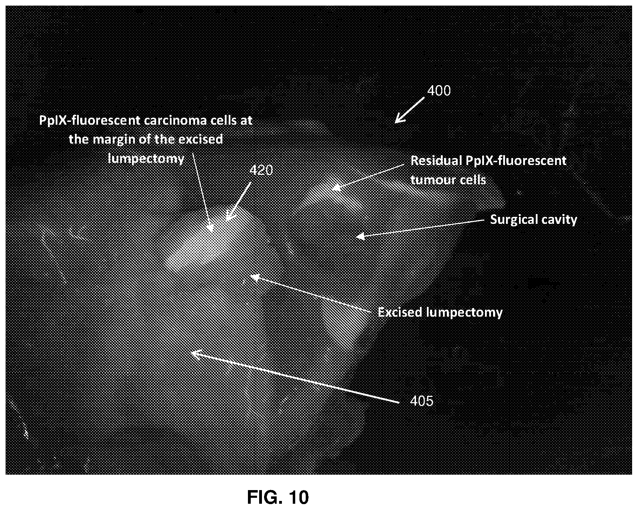

In accordance with another aspect of the present disclosure, tissue phantoms may incorporate diseased cells in the "diseased" portion of the phantom. For example, in one example embodiment of a breast tissue phantom, the portion of the phantom configured to mimic cancerous tissue comprises carcinoma cells. In preparing this phantom, carcinoma cells were treated with 5-ALA, resuspended in tissue phantom material and injected under the surface of a normal or healthy tissue phantom to create a "tumor." The tumor was resected from the breast tissue phantom, creating an excised tissue specimen or phantom lumpectomy. The phantom lumpectomy and the surgical cavity were examined using widefield fluorescence imaging. Residual carcinoma cells producing PpIX were visualized in the surgical cavity and at the margins of the excised lumpectomy. The image of FIG. 10 illustrates an example embodiment of a breast tissue phantom 400 comprising carcinoma cells in the diseased tissue portion 420 of the phantom. The PpIX-containing carcinoma cells and phantom material may be injected into the healthy phantom tissue 405 to form discrete diseased portions or tumors 420 in phantom 400.

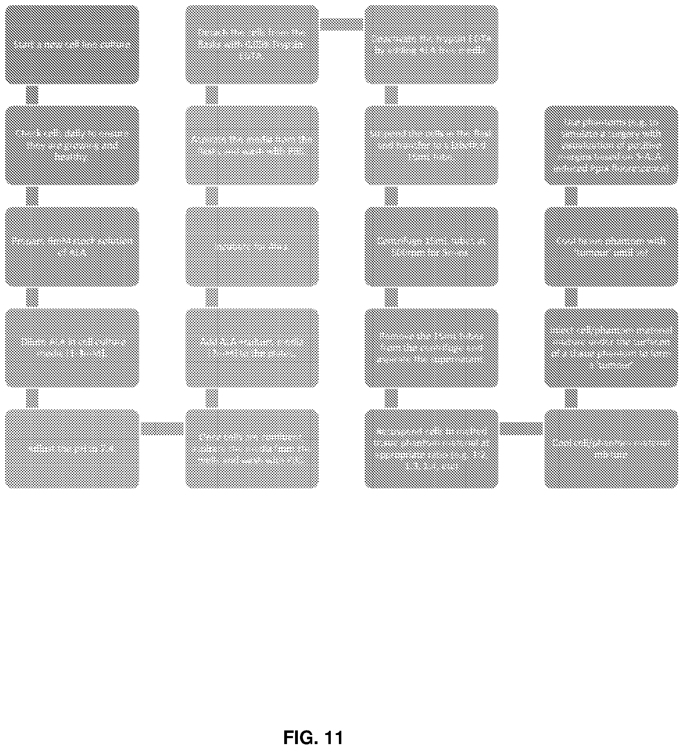

An example process of making the carcinoma tumors for inclusion in the healthy tissue of the tissue phantom is described below and shown in the flowchart of FIG. 11. Example cells lines that may be used include MDA-MB-468 (ATCC^{®} HTB-132^{™}); MDA-MB-231 (ATCC^{®} CRM-HTB-26^{™}); MCF-7(ATCC^{®} HTB-22^{™}); BxPC3 (ATCC^{®} CRL-1687^{™}); and C6 (ATCC^{®} CCL-107^{™}).

### Protocol

### Prep

### Cells

1. Supplement new media (RPMI or DMEM) with 10% heat-inactivated fetal bovine serum, penicillin (0.05 mg/ml) and streptomycin (0.05 mg/ml).
2. Start a new cell line culture from liquid nitrogen stored cryovials in T25 plate
   2.1. Use complete supplemented RPMI media for BxPC3 cell line.
   2.2. Use complete supplemented DMEM for MDA-MB-468, MDA-MB-231, C6, and MCF-7 cell lines.
3. Check cells daily to ensure they are growing and healthy. Follow ATCC recommendations for splitting cells into T75 Flasks.

### 5-ALA

1. Prepare 6mM stock solution of ALA by dissolving 15.736mg of 5-ALA in 20mL of PBS.
2. For each T75 plate of BXPC3 cells, add 2mL of stock solution of ALA with 4mL of warmed complete RPMI media [2mM]
3. For each T75 plate of the other cell lines, add 2mL of stock solution of ALA with 4mL of warmed complete DMEM media [2mM].
4. Test the pH of the adjusted media to ensure that the pH is 7.4.

### Treatment

1. Once cells are between 70-90% confluent in a T75 plate, aspirate the media from the wells and wash with 2mL of PBS.
2. Add 6mL of appropriate ALA media to the plates.
3. Incubate for 4hrs.
4. Keeping the Biosafety cabinet dark, aspirate the media from the flasks and wash with 2mL of PBS.
5. Detach the cells from the flasks with 2mL of warmed 0.05% Trypsin-EDTA. Wait 1-3 minutes for the cells to detach, the BxPC3 cells may require additional time. Monitor flasks closely to ensure that they do not sit in trypsin-EDTA for more time than necessary.
6. Deactivate the trypsin EDTA by adding 2mL of warmed complete ALA-free media.
7. Suspend the cells in the flask and transfer to a labelled 15mL tube.
8. Centrifuge 15mL tubes at 500rpm for 5mins.
9. Resuspend the cells in 5mL of PBS.
10. Count cells
   a) remove 10ul of each tube and place in individual microfuge tubes.
   b) dilute with 10ul of trypan blue.
   c) place 10uL in the hemocytometer
   d) count cells
11. Centrifuge each 15mL tube at 500rpm for 5mins.
12. Remove the 15mL tubes from the centrifuge and aspirate the supernatant.

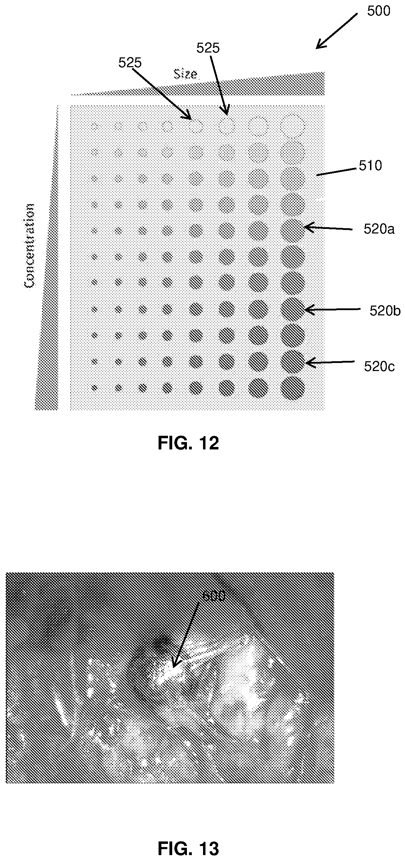

In accordance with another aspect of the present disclosure tissue phantoms may be used to determine a depth of healthy tissue through which diseased tissue can be detected, for example by an imaging device as described herein. A depth tissue phantom 500 is illustrated in FIG. 12. In accordance with the present teachings, a depth phantom 500 is a phantom that has been designed to measure the fluorescence limit of detection of an imaging device in terms of depth, target size, and target concentration. This phantom 500 comprises a microfluidics chip 510 preloaded with PpIX at varying concentrations 520a, 520b, 520c, etc. The size of the wells 525 that contain the PpIX can vary within any range. An example range is 10 µm-1 mm. The chip is placed in a tray and imaged. Then, a small amount of the phantom material designed for 405 nm wavelength excitation is poured to cover the chip. Once the phantom material sets, the chip is imaged again. This process would be repeated until no more fluorescence is visible from any of the wells at some depth.

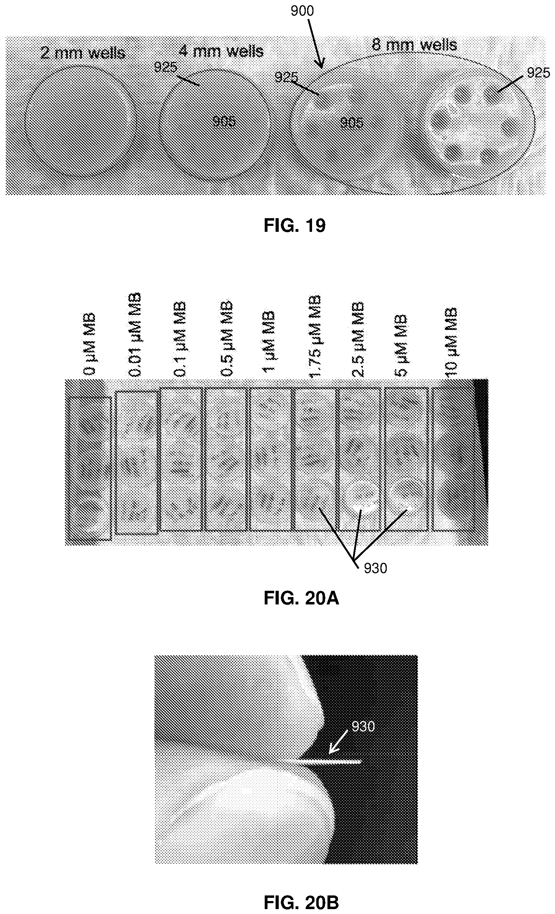

As an alternative to pouring phantom material over the chip, one or more thin film phantoms may be used. The phantom material can be used to create thin films with thickness around 100 µm or less, by dehydrating the liquid phantom material. This material can be used to mimic thin films of tissue to measure the depth below the tissue surface at which a fluorophore can still be detected using, for example, a depth phantom. See, for example, FIGS. 20A and 20B.

In accordance with another aspect of the present disclosure, a tissue phantom may be used to identify potential interactions between different types of imaging and/or contrast agents used together during surgery, such as BCS, to make residual cancer cells, precancerous cells, satellite lesions, and/or other malignant cells in surgical cavities, tissue specimens, lymph nodes, or other areas visible to the surgeon.

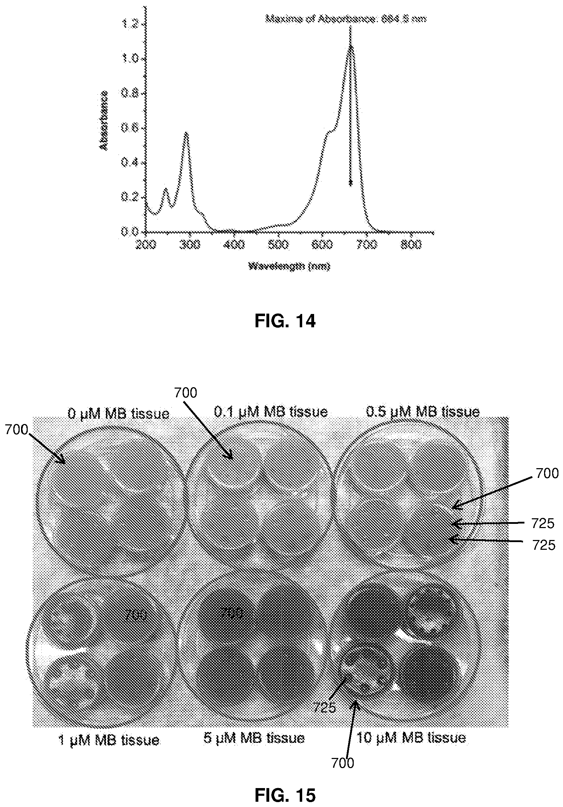

During breast cancer surgery, surgeons may perform a biopsy on the sentinel lymph node(s) 600 surrounding the tumor. By using methods such as blue dye localization or radioactive tracers, surgeons can locate the sentinel lymph nodes to dissect them and determine if the cancer has metastasized from the original tumor. In breast cancer surgery, typically, the blue dye is subcutaneously injected in the tumor, the surrounding tissue (peri-tumor), or near the nipple, so that it may diffuse through the lymphatic vessels and reach the nearby sentinel lymph nodes, where it accumulates to turn blue. This allows surgeons to visualize the sentinel lymph nodes using conventional white lighting and the unaided eye. An example of this is shown in the image of FIG. 13. One of the most common dyes for surgical purposes is Methylene Blue (MB). MB absorbs certain wavelengths of light, often in the 600-700 nm range. The peak absorption of MB is 665 nm as shown in FIG. 14.

MB is typically administered in 3-5 mL volumes at a 1% concentration. It is suspected that MB has a strong ability to absorb light in the 550-700 nm wavelength spectrum. Under 405 nm excitation, protoporphyrin IX's (PpIX's) peak emission occurs at ~635 nm, as shown in FIG. 1B, which overlaps with the absorption spectra of MB (or blue dye), potentially attenuating the PpIX fluorescence (FL) emission due to the spectral overlap. This spectral overlap is well known in the field of intraoperative fluorescence imaging, however, there is a paucity of information about this issue in the area of (fluorescence imaging) during breast cancer surgery.

Tissue phantoms in accordance with the present disclosure can be used to obtain a better understanding of this phenomenon to better appreciate its potential effect (if any) on the fluorescence detection of (tumor) PpIX fluorescence when MB (or similar blue dye) is used intraoperatively for SLN detection during breast cancer surgery.

The phantom material "recipe" can be modified to incorporate dyes, fluorophores, or other materials directly into the phantom material. In one example embodiment, a plurality of tissue phantoms were created, the phantoms having increasing concentrations of Methylene Blue (MB) in the phantom tissue material. A plurality of wells or recesses were then created in each tissue phantom. The wells in the phantoms were also created with punch biopsy tools although other methods can be used. In these wells, a mixture of PpIX, methylene blue, and phantom material was pipetted to form solid phantoms.

To create the MB tissue phantom, MB and PpIX were diluted in phantom material (PM), which was prepared as described below. When mixing PM with PpIX, for longevity and reproducibility, a proportion of PM >75% allows the mixture to solidify and minimizes the liquid content being absorbed by surrounding PM. A combination of PpIX diluted with PM to 5 µM PpIX and 90% PM proportion produces bright red FL when imaged.

As previously described with respect to other embodiments of a tissue phantom, the phantom material comprises two mixtures: a tris buffer and gelatin mixture. In preparing MB tissue phantoms, the formulation was adjusted to include methylene blue by combining cooled base phantom mixture with concentrated stocks of 100, 50, 10, 5, 1, and 0 µM methylene blue, to dilute by a factor of 10, which achieved a final concentration of 10, 5, 1, 0.5, 0.1, and 0 µM methylene blue phantom mixture. These mixtures were used to create 25 mL phantom samples with a gradient of methylene blue concentrations. Three different MB phantom models were created.

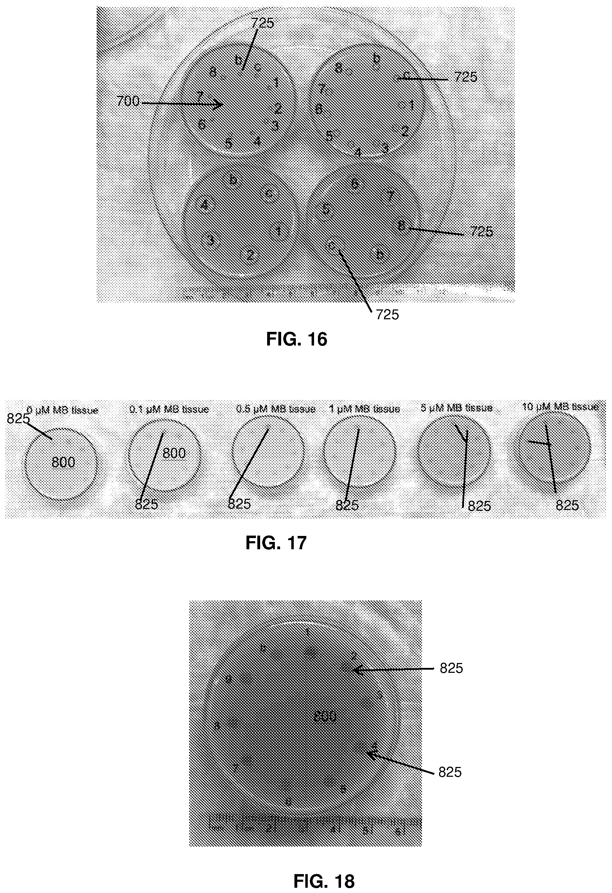

The first MB tissue phantom is designed to simulate the clinical scenario where, at the surgical margin, there is residual tumor (containing PpIX) that also contains MB within the tumor, and/or is surrounded by normal tissue containing MB. This first MB tissue phantom was used to measure which MB concentrations (in the tissue and within the tumor) are able to decrease the FL signal from a single concentration of PpIX. The phantoms 700 were designed as pucks, each with ten wells 725 cut with a punch biopsy tool. This was repeated for 3 punch biopsy sizes: 2 mm, 4 mm and 8 mm (The 8 mm phantoms had to be split into two "pucks" due to the relative size of the wells and the pucks, FIG. 16). Six groups of these phantoms were made, one with each of the following concentrations of MB mixed into the phantom tissue: 0, 0.1, 0.5, 1, 5, 10 µM (FIGS. 15 and 16). Pucks measured 55 mm in diameter and 10 mm in thickness. The wells were filled as follows (the %PM is the proportion of the mixture that is PM) and allowed to set until solidified:
- b: PM - represents background tissue phantom
- c: 5 µM PpIX + water + 89% PM
- 1: 5 µM PpIX + 0.01 µM MB + 89% PM
- 2: 5 µM PpIX + 0.1 µM MB + 89% PM
- 3: 5 µM PpIX + 0.5 µM MB + 89% PM
- 4: 5 µM PpIX + 1 µM MB+ 89% PM
- 5: 5 µM PpIX + 1.75 µM MB+ 89% PM
- 6: 5 µM PpIX + 2.5 µM MB + 89% PM
- 7: 5 µM PpIX + 5 µM MB + 89% PM
- 8: 5 µM PpIX + 10 µM MB + 89% PM

Each phantom was imaged under FL imaging using an imaging device as described herein to determine the imaging effect of each concentration of MB on PpIX fluorescence.

A second MB tissue phantom 800 was created to measure how the FL from various concentrations of PpIX is affected by a given concentration of MB. Examples of these tissue phantoms 800 are shown in FIGS. 17 and 18. Six versions of the second MB tissue phantoms were made with the same variation in MB tissue concentration as the first MB tissue phantom. The ten wells 825 cut in these phantoms all contain phantom tissue material mixture and a concentration of MB which matches the tissue, with one of nine PpIX concentrations. The wells were filled as follows (the %PM is the proportion of the mixture that is PM):
- b: PM + MB (same [MB] as tissue) - represents background tissue phantom
- 1: 50 µM PpIX + MB + 89% PM (same [MB] as tissue)
- 2: 25 µM PpIX + MB + 89% PM (same [MB] as tissue)
- 3: 10 µM PpIX + MB + 89% PM (same [MB] as tissue)
- 4: 5 µM PpIX + MB + 89% PM (same [MB] as tissue)
- 5: 2.5 µM PpIX + MB + 89% PM (same [MB] as tissue)
- 6: 1 µM PpIX + MB + 89% PM (same [MB] as tissue)
- 7: 0.5 µM PpIX + MB + 89% PM (same [MB] as tissue)
- 8: 0.25 µM PpIX + MB + 89% PM (same [MB] as tissue)
- 9: 0.1 µM PpIX + MB + 89% PM (same [MB] as tissue)

A third MB tissue phantom 900 was made to test the effect of a thin layer of MB-containing tissue phantom covering normal tissue and tumor that do not contain MB. The third MB tissue phantom comprises a first part 905 - a tissue phantom that does not contain MB and a second part comprising a thin film MB tissue phantom 930 similar to the thin film tissue phantoms described above. Each first part tissue phantom 905 has ten wells 925 cut, all identically filled with 5 µM PpIX and 89% PM (see FIG. 18). The second part of the third MB tissue phantom 900 is a thin dehydrated MB tissue phantom film 930 (FIGS. 20A and 20B) which were produced, each with one of nine concentrations of MB. The films 930 were made by filling a 58-mm diameter dish with 3 mL of PM with the desired concentration of MB and allowing to dehydrate at 21C, ~55% humidity for 48 hours. These films are 100 +/- 25 µm in thickness as measured with a Mitutoyo Digimatic Micrometer 293-832-30. To replicate the scenario where a thin layer of MB is on the surface of a tumor with PpIX, a "pizza slice" shape was cut from each film and placed over a portion of the phantom puck, fully covering a well.

The MB tissue phantoms discussed herein were used to test the effect of blue dyes on PpIX, with the concentrations of the blue dye and PpIX both varied using different phantom models to isolate the impact of each concentration of blue dye on each concentration of PpIX. Such an experiment can also be conducted using phantoms that replace with PpIX with some other FL contrast agent such as ICG, IRDye800, or fluorescein. The blue dye can be replaced with another (clinically relevant) dye (such as for example Patent Blue V, Indigo Carmine, Isosulfan Blue, and specimen inking dyes such as, for example, India ink or other proprietary formulations of specimen inking dyes) to understand the interaction of the standard of care dye/material on the desired fluorescence signal.

It will be appreciated by those ordinarily skilled in the art having the benefit of this disclosure that the present disclosure provides various exemplary devices, systems, and methods for tumor visualization. Further modifications and alternative embodiments of various aspects of the present disclosure will be apparent to those skilled in the art in view of this description.

Furthermore, the devices and methods may include additional components or steps that were omitted from the drawings for clarity of illustration and/or operation. Accordingly, this description is to be construed as illustrative only and is for the purpose of teaching those skilled in the art the general manner of carrying out the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present disclosure may be utilized independently, all as would be apparent to one skilled in the art after having the benefit of the description herein. Changes may be made in the elements described herein without departing from the scope of the present disclosure.

It is to be understood that the particular examples and embodiments set forth herein are non-limiting, and modifications to structure, dimensions, materials, and methodologies may be made without departing from the scope of the present disclosure.

Furthermore, this description's terminology is not intended to limit the present disclosure. For example, spatially relative terms-such as "beneath," "below," "lower," "above," "upper," "bottom," "right," "left," "proximal," "distal," "front," and the like-may be used to describe one element's or feature's relationship to another element or feature as illustrated in the figures. These spatially relative terms are intended to encompass different positions (i.e., locations) and orientations (i.e., rotational placements) of a device in use or operation in addition to the position and orientation shown in the drawings.

For the purposes of this specification, unless otherwise indicated, all numbers expressing quantities, percentages or proportions, and other numerical values used in the specification are to be understood as being modified in all instances by the term "about" if they are not already. Accordingly, unless indicated to the contrary, the numerical parameters set forth in the following specification are approximations that may vary depending upon the desired properties sought to be obtained by the present disclosure.

Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all sub-ranges subsumed therein.

It is noted that, as used in this specification the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

It should be understood that while the present disclosure has been described in detail with respect to various exemplary embodiments thereof, it should not be considered limited to such, as numerous modifications are possible without departing from the scope of the appended claims.

## Claims

1. A tissue phantom, comprising:
a first portion (105) having the optical properties of healthy tissue, wherein the first portion (105) comprises one or more of the following: tris buffer, gelatin, agar, silicone and organic tissues from humans or animals; and
a second portion (120) having the optical properties of diseased or abnormal tissue, optionally of cancerous tissue, the second portion (120) including a plurality of regions,
**characterized in that** each region includes a different concentration of a material configured to fluoresce in response to illumination by excitation light.

2. The tissue phantom of claim 1, wherein the optical properties of the healthy tissue are substantially the same as the optical properties of healthy breast tissue, and/or wherein the optical properties of the diseased or abnormal tissue, optionally of cancerous tissue, are substantially the same as the optical properties of cancerous breast tissue.

3. The tissue phantom of any one of claim 1 or 2, wherein the first portion (105) has the optical properties of healthy tissue when illuminated with excitation light having a wavelength of about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, and/or combinations thereof.

4. The tissue phantom of any one of claims 1-3, wherein the first portion (105) has the optical properties of healthy tissue when illuminated with excitation light having a wavelength of about 400 nm to about 450 nm, or a wavelength of about 405 nm ± 10 nm.

5. The tissue phantom of any one of claims 1-4, wherein the first portion (105) has the optical properties of healthy tissue when illuminated with excitation light having a wavelength of about 750 nm - 800 nm.

6. The tissue phantom of any one of claims 1-5, wherein the second portion (120) has the optical properties of cancerous tissue when illuminated with excitation light having a wavelength of about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, and/or combinations thereof,
optionally wherein the second portion (120) has the optical properties of cancerous tissue when illuminated with excitation light having a wavelength of about 400 nm to about 450 nm.

7. The tissue phantom of any one of claim 1-6, wherein the excitation light has a wavelength of about 405 nm ± 10 nm, about 760 nm ± 10 nm, about 770 nm ± 10 nm, or about 780 nm ± 10 nm.

8. The tissue phantom of any one of claims 1-7, wherein the second portion (120) has the optical properties of vasculature or microvasculature when illuminated with excitation light having a wavelength of about 750 nm - 800 nm.

9. The tissue phantom of any one of claims 1-8, wherein the fluorescence emission of each of the plurality of regions varies in intensity based on the respective concentration of the material configured to fluoresce in response to illumination by excitation light.

10. The tissue phantom of any one of claims 1-9, wherein an optical response of the first portion (105) is distinguishable from the optical response of the second portion (120) when illuminated with the same excitation light due to differences between optical properties of the first and second portions.

11. The tissue phantom of any one of claims 1-10, wherein the second portion (120) contains one or more of protoporphyrin IX (PpIX), indocyanine green (ICG), Methylene Blue (MB), a fluorescent dye, IRDye 800CW, cyanine fluorescent dyes, AlexaFluor dyes, or other fluorophores of interest.

12. The tissue phantom of any one of claims 1-11, wherein the second portion (120) contains carcinoma cells.

13. The tissue phantom of any one of claims 1-12, wherein the optical properties of the first portion (105) of the tissue phantom vary no more than 2% throughout the first portion.

14. The tissue phantom of any one of claims 1-13, wherein the tissue phantom is molded to a form of a body part, a human breast, or an excised breast tissue specimen.

15. The tissue phantom of any one of claims 1-14, wherein the second portion (120) has the optical properties of cancerous tissue.

## Patentansprüche

1. Gewebephantom, umfassend:
einen ersten Abschnitt (105), welcher die optischen Eigenschaften von gesundem Gewebe aufweist, wobei der erste Abschnitt (105) eines oder mehrere der folgenden umfasst: Tris-Puffer, Gelatine, Agar, Silikon und organische Gewebe von Menschen oder Tieren; und
einen zweiten Abschnitt (120), welcher die optischen Eigenschaften von abgestorbenem oder anormalem Gewebe aufweist, optional von Krebsgewebe, wobei der zweite Abschnitt (120) eine Mehrzahl von Regionen umfasst,
**dadurch gekennzeichnet, dass** jede Region eine unterschiedliche Konzentration eines Materials umfasst, welches dazu eingerichtet ist, in Reaktion auf eine Beleuchtung durch Anregungslicht zu fluoreszieren.

2. Gewebephantom nach Anspruch 1, wobei die optischen Eigenschaften des gesunden Gewebes im Wesentlichen dieselben sind wie die optischen Eigenschaften von gesundem Brustgewebe und/oder wobei die optischen Eigenschaften des abgestorbenen oder anormalen Gewebes, optional von Krebsgewebe, im Wesentlichen dieselben sind wie die optischen Eigenschaften von Brustkrebsgewebe.

3. Gewebephantom nach einem der Ansprüche 1 oder 2, wobei der erste Abschnitt (105) die optischen Eigenschaften von gesundem Gewebe aufweist, wenn er mit Anregungslicht beleuchtet wird, welches eine Wellenlänge von etwa 350 nm - etwa 400 nm, etwa 400 nm - etwa 450 nm, etwa 450 nm - etwa 500 nm, etwa 500 nm - etwa 550 nm, etwa 550 nm - etwa 600 nm, etwa 600 nm - etwa 650 nm, etwa 650 nm - etwa 700 nm, etwa 700 nm - etwa 750 nm, etwa 750 nm - etwa 800 nm, etwa 800 nm - etwa 850 nm, etwa 850 nm - etwa 900 nm und/oder Kombinationen daraus aufweist.

4. Gewebephantom nach einem der Ansprüche 1-3, wobei der erste Abschnitt (105) die optischen Eigenschaften von gesundem Gewebe aufweist, wenn er mit Anregungslicht beleuchtet wird, welches eine Wellenlänge von etwa 400 nm bis etwa 450 nm oder eine Wellenlänge von etwa 405 nm ± 10 nm aufweist.

5. Gewebephantom nach einem der Ansprüche 1-4, wobei der erste Abschnitt (105) die optischen Eigenschaften von gesundem Gewebe aufweist, wenn er mit Anregungslicht beleuchtet wird, welches eine Wellenlänge von etwa 750 nm - 800 nm aufweist.

6. Gewebephantom nach einem der Ansprüche 1-5, wobei der zweite Abschnitt (120) die optischen Eigenschaften von Krebsgewebe aufweist, wenn er mit Anregungslicht beleuchtet wird, welches eine Wellenlänge von etwa 350 nm - etwa 400 nm, etwa 400 nm - etwa 450 nm, etwa 450 nm - etwa 500 nm, etwa 500 nm - etwa 550 nm, etwa 550 nm - etwa 600 nm, etwa 600 nm - etwa 650 nm, etwa 650 nm - etwa 700 nm, etwa 700 nm - etwa 750 nm, etwa 750 nm - etwa 800 nm, etwa 800 nm - etwa 850 nm, etwa 850 nm - etwa 900 nm und/oder Kombinationen daraus aufweist;
wobei optional der zweite Abschnitt (120) die optischen Eigenschaften von Krebsgewebe aufweist, wenn er mit Anregungslicht beleuchtet wird, welches eine Wellenlänge von etwa 400 nm bis etwa 450 nm aufweist.

7. Gewebephantom nach einem der Ansprüche 1-6, wobei das Anregungslicht eine Wellenlänge von etwa 405 nm ± 10 nm, etwa 760 nm ± 10 nm, etwa 770 nm ± 10 nm oder etwa 780 nm ± 10 nm aufweist.

8. Gewebephantom nach einem der Ansprüche 1-7, wobei der zweite Abschnitt (120) die optischen Eigenschaften von Blutgefäßen oder Mikro-Blutgefäßen aufweist, wenn er mit Anregungslicht beleuchtet wird, welches eine Wellenlänge von etwa 750 nm - 800 nm aufweist.

9. Gewebephantom nach einem der Ansprüche 1-8, wobei die Fluoreszenz-Emission von jeder aus der Mehrzahl von Regionen in ihrer Intensität auf Grundlage der jeweiligen Konzentration des Materials variiert, welches dazu eingerichtet ist, in Reaktion auf eine Beleuchtung durch Anregungslicht zu fluoreszieren.

10. Gewebephantom nach einem der Ansprüche 1-9, wobei eine optische Reaktion des ersten Abschnitts (105) von der optischen Reaktion des zweiten Abschnitts (120) unterscheidbar ist, wenn sie mit demselben Anregungslicht beleuchtet werden, aufgrund von Unterschieden zwischen optischen Eigenschaften der ersten und zweiten Abschnitte.

11. Gewebephantom nach einem der Ansprüche 1-10, wobei der zweite Abschnitt (120) eines oder mehrere aus Protoporphyrin IX (PpIX), Indocyanin-Grün (ICG), Methylen-Blau (MB), einem fluoreszierenden Farbstoff, IRDye 800CW, Cyanin-Fluoreszenzfarbstoffen, AlexaFluor-Farbstoffen oder anderen Fluorophoren von Interesse umfasst.

12. Gewebephantom nach einem der Ansprüche 1-11, wobei der zweite Abschnitt (120) Karzinom-Zellen umfasst.

13. Gewebephantom nach einem der Ansprüche 1-12, wobei die optischen Eigenschaften des ersten Abschnitts (105) des Phantomgewebes um nicht mehr als 2% über den ersten Abschnitt variieren.

14. Gewebephantom nach einem der Ansprüche 1-13, wobei das Phantomgewebe zu einer Form eines Körperteils, einer menschlichen Brust oder einer exzidierten Brustgewebe-Probe geformt ist.

15. Gewebephantom nach einem der Ansprüche 1-14, wobei der zweite Abschnitt (120) die optischen Eigenschaften von Krebsgewebe aufweist.

## Revendications

1. Fantôme tissulaire, comprenant :
une première portion (105) ayant les propriétés optiques de tissu sain, dans lequel la première portion (105) comprend l'un ou plusieurs de ce qui suit : tampon tris, gélatine, agar-agar, silicone et tissus organiques provenant d'êtres humains ou animaux ; et
une seconde portion (120) ayant les propriétés optiques de tissu malade ou anormal, éventuellement de tissu cancéreux, la seconde portion (120) incluant une pluralité de régions,
**caractérisé en ce que** chaque région inclut une concentration différente d'un matériau configuré pour émettre une fluorescence en réponse à un éclairage par de la lumière d'excitation.

2. Fantôme tissulaire selon la revendication 1, dans lequel les propriétés optiques du tissu sain sont sensiblement identiques aux propriétés optiques du tissu sain de sein, et/ou dans lequel les propriétés optiques du tissu malade ou anormal, éventuellement de tissu cancéreux, sont sensiblement identiques aux propriétés optiques du tissu cancéreux de sein.

3. Fantôme tissulaire selon l'une quelconque des revendications 1 ou 2, dans lequel la première portion (105) présente les propriétés optiques de tissu sain lorsqu'éclairée avec de la lumière d'excitation ayant une longueur d'onde d'environ 350 nm - environ 400 nm, environ 400 nm - environ 450 nm, environ 450 nm - environ 500 nm, environ 500 nm - environ 550 nm, environ 550 nm - environ 600 nm, environ 600 nm - environ 650 nm, environ 650 nm - environ 700 nm, environ 700 nm - environ 750 nm, environ 750 nm - environ 800 nm, environ 800 nm - environ 850 nm, environ 850 nm - environ 900 nm, et/ou leurs combinaisons.

4. Fantôme tissulaire selon l'une quelconque des revendications 1 à 3, dans lequel la première portion (105) présente les propriétés optiques du tissu sain lorsqu'éclairée avec de la lumière d'excitation ayant une longueur d'onde d'environ 400 nm à environ 450 nm, ou une longueur d'onde d'environ 405 nm ± 10 nm.

5. Fantôme tissulaire selon l'une quelconque des revendications 1 à 4, dans lequel la première portion (105) présente les propriétés optiques de tissu sain lorsqu'éclairée avec de la lumière d'excitation ayant une longueur d'onde d'environ 750 nm à 800 nm.

6. Fantôme tissulaire selon l'une quelconque des revendications 1 à 5, dans lequel la seconde portion (120) présente les propriétés optiques de tissu cancéreux lorsqu'éclairée avec de la lumière d'excitation ayant une longueur d'onde d'environ 350 nm - environ 400 nm, environ 400 nm - environ 450 nm, environ 450 nm - environ 500 nm, environ 500 nm - environ 550 nm, environ 550 nm - environ 600 nm, environ 600 nm - environ 650 nm, environ 650 nm - environ 700 nm, environ 700 nm - environ 750 nm, environ 750 nm - environ 800 nm, environ 800 nm - environ 850 nm, environ 850 nm - environ 900 nm, et/ou leurs combinaisons ;
éventuellement dans lequel la seconde portion (120) présente les propriétés optiques de tissu cancéreux lorsqu'éclairée avec de la lumière d'excitation ayant une longueur d'onde d'environ 400 nm à environ 450 nm.

7. Fantôme tissulaire selon l'une quelconque des revendications 1 à 6, dans lequel la lumière d'excitation présente une longueur d'onde d'environ 405 nm ± 10 nm, environ 760 nm ± 10 nm, environ 770 nm ± 10 nm, ou environ 780 nm ± 10 nm.

8. Fantôme tissulaire selon l'une quelconque des revendications 1 à 7, dans lequel la seconde portion (120) présente les propriétés optiques de vasculature ou de microvasculature lorsqu'éclairée avec de la lumière d'excitation ayant une longueur d'onde d'environ 750 nm à 800 nm.

9. Fantôme tissulaire selon l'une quelconque des revendications 1 à 8, dans lequel l'émission de fluorescence de chacune de la pluralité de régions varie en intensité sur la base de la concentration respective du matériau configuré pour émettre une fluorescence en réponse à l'éclairage par de la lumière d'excitation.

10. Fantôme tissulaire selon l'une quelconque des revendications 1 à 9, dans lequel une réponse optique de la première portion (105) peut être distinguée de la réponse optique de la seconde portion (120) lorsqu'éclairée avec la même lumière d'excitation due à des différences entre des propriétés optiques de la première et de la seconde portion.

11. Fantôme tissulaire selon l'une quelconque des revendications 1 à 10, dans lequel la seconde portion (120) contient l'un ou plusieurs parmi la protoporphyrine IX (PpIX), le vert d'indocyanine (ICG), le bleu de méthylène (MB), un colorant fluorescent, le colorant IR 800CW, les colorants fluorescents de cyanine, les colorants AlexaFluor, ou d'autres fluorophores d'intérêt.

12. Fantôme tissulaire selon l'une quelconque des revendications 1 à 11, dans lequel la seconde portion (120) contient des cellules de carcinome.

13. Fantôme tissulaire selon l'une quelconque des revendications 1 à 12, dans lequel les propriétés optiques de la première portion (105) du fantôme tissulaire varient de pas plus de 2 % à travers la première portion.

14. Fantôme tissulaire selon l'une quelconque des revendications 1 à 13, dans lequel le fantôme tissulaire est moulé selon une forme d'une partie corporelle, d'un sein humain, ou d'un échantillon de tissu de sein excisé.

15. Fantôme tissulaire selon l'une quelconque des revendications 1 à 14, dans lequel la seconde portion (120) présente les propriétés optiques de tissu cancéreux.
